# EUROPEAN PATENT SPECIFICATION

(11) **EP 3 024 375 B1**
(45) Date of publication and mention of the grant of the patent: **01.09.2021**
(21) Application number: 14829823.5
(22) Date of filing: 21.07.2014
(51) Int. Cl.: A61B 1/04, A61M 16/00, A61M 16/04, A61B 7/00, A61B 1/00, A61B 1/05, A61B 1/012, A61B 1/06, A61B 1/267

(54) **MEDICAL DEVICE, AND THE METHODS OF USING SAME**
MEDIZINISCHE VORRICHTUNG UND VERFAHREN ZUR VERWENDUNG DAVON
DISPOSITIF MÉDICAL ET SES MÉTHODES D'UTILISATION

(30) Priority: 22.07.2013 US 201313947610
(43) Date of publication of application: 01.06.2016
(73) Proprietor: WM & DG, Inc., Deerfield, IL 60015 (US)
(72) Inventor: MOLNAR, Robert, Long Grove, IL 60047 (US)
(74) Representative: FRKelly
(86) International application number: PCT/US2014/047385
(87) International publication number: WO 2015/013172

(56) References cited:
- US-A- 4 360 008
- US-A- 4 584 998
- US-A1- 2005 182 297
- US-A1- 2007 137 651
- US-A1- 2007 137 651
- US-A1- 2008 021 273
- US-A1- 2010 249 639
- US-A1- 2011 178 372
- US-A1- 2011 178 372
- US-A1- 2011 201 882
- US-A1- 2012 260 921
- US-A1- 2012 302 833
- US-A1- 2013 030 249
- US-A1- 2013 030 249
- US-B1- 6 443 156
- US-B2- 7 156 091
- None

## Description

### FIELD OF THE INVENTION

The present invention relates to a medical device for allowing a medical professional to see the internal membranes of a patient during a medical procedure.

### BACKGROUND OF THE INVENTION

An example of a medical device which medical professionals use to see the internal membranes of a patient is a laryngeal mask airway. A laryngeal mask airway is used to ventilate and to supply anesthetic to a patient during surgery. A laryngeal mask airway is different than an endotracheal tube in that the laryngeal mask airway is positioned in the throat of the patient proximally of the vocal folds, while an endotracheal tube is passed through the vocal folds and is positioned in the patient's trachea.

Laryngeal mask airways of the prior art generally have a tube opening into the center of a generally elliptical dome. The tube is generally straight, but can flex to assume a curved shape. A cuff, which may be inflatable, is sometimes attached to the perimeter of the dome.

In use, the medical professional inserts the laryngeal mask airway into the mouth of the patient. The open tube allows the patient to breathe on his/her own during insertion. The tube can also be connected to a ventilator to provide assisted breathing to the patient. For insertion, the cuff (if provided), the dome and the tube slide against the hard palate and then against the soft palate and into the pharynx of the patient. This procedure is performed blindly and only by feel which comes from experience in performing the procedure. Trauma to the patient may occur when placing the laryngeal mask airway as a result of the laryngeal mask airway attempting to conform to a curved position in the pharynx. When properly positioned in the hypo-pharynx, the proximal end of the cuff seats against the epiglottis pushing it toward the tongue of the patient and the distal end of the cuff seats in the esophagus. At times, the cuff may be positioned such that the epiglottis is pushed downwardly and may at least partially block the tube opening. This is not a desirable result as the blocking by the epiglottis can cause problems with the airflow through the laryngeal mask airway. In addition, inappropriate sizing and differences in the anatomy of patients may also impair the proper positioning of the laryngeal mask airway. Since the insertion is performed blindly, the medical professional will not know if proper placement of the laryngeal mask airway has occurred. After positioning the laryngeal mask airway, the inflatable cuff (if provided) is inflated and the patient's esophagus is blocked by the cuff. The medical professional will listen for breath sounds and ascertain end tidal CO₂ gases from the patient to verify proper positioning of the laryngeal mask airway.

If the medical professional needs to insert an endotracheal tube into the patient, the endotracheal tube can be inserted through the tube of the laryngeal mask airway to intubate the patient. If the epiglottis is at least partially blocking the opening in the tube, this intubation may be difficult. In addition, the glottis opening quite often does not align with the tube opening which can make this blind insertion difficult and may result in trauma to the laryngeal inlet.

US 2011/0178372 A1 discloses video-based laryngoscopes having an external centrally located channel coursing from the handle to a curved blade configured to deliver an ETT to a trachea as visualized by a non-removable, or alternatively, a removable video camera and lighting unit located beneath the external channel. The channel laryngoscope may be made for single use in a patient and discarded, or be processed for re-use. Alternatively, the channel laryngoscope may have a removable video camera and lighting member located within an internal chamber that runs parallel with the external channel. In another aspect, a laryngoscope adapter may be detachably affixable to the channel laryngoscope equipped with the non-removable camera to provide a decontaminated or sterile surface to permit re-use of the channel laryngoscope without having to undergo washing or decontamination procedures.

US 2012/0302833 A1 discloses an intubation system including a tracheal tube and an illumination assembly that is removably couplable to a tubular body of the tracheal tube. The tracheal tube may be a double lumen tracheal tube having a first cuff that is adapted to be inflated to seal against the walls of a patient's trachea and a second cuff that is adapted to be inflated to seal against the walls of the patient's bronchial stem. The illumination assembly may have one or more illumination devices that are adapted to produce light within the patient's trachea, the patient's bronchial stem, or both when the illumination assembly is coupled to the tubular body.

US 2007/0137651 A1 discloses an airway apparatus and method of use, in which the airway device includes a dual lumen airway having imaging apparatus, self-inflating balloons, and other sensors, thereby allowing rapid intubation and ventilation.

US 2013/0030249 A1 discloses systems, methods, and devices for facilitating insertion of an endotracheal tube and/or for verifying the position of the endotracheal tube within an airway of a patient with respect to an anatomical landmark of a patient. Systems, methods, and devices for facilitating removal of debris from the distal airways of a patient under direct visualization are also disclosed.

US 2010/249639 A1 discloses an airway management device including: an endotracheal tube having a proximal end and a distal end and a sheath adjacent to the endotracheal tube. The sheath includes a proximal end and a distal end. The distal end of the endotracheal tube extends beyond the distal end of the sheath. The device further comprises a non-removable camera at the distal end of the sheath.

A medical device is provided herein which provides improvements to existing laryngeal mask airways and which overcomes the disadvantages presented by the prior art. Other features and advantages will become apparent upon a reading of the attached specification, in combination with a study of the drawings.

### SUMMARY OF THE INVENTION

A medical device is provided for insertion into a cavity of a patient to visual the internal membranes of the patient. The medical device can be an endotracheal tube, a suction tube, a bronchoscope, a tube changer, an esophageal tube, an intubating tube, an esophageal tube in combination with a separate intubating tube, a device for manipulating the position of the epiglottis of the patient, a stylet, or a tube insertable into the vagina of the patient. The medical device has a camera lumen having a sealed window at one end thereof attached thereto, and a separate camera which is insertable into the camera lumen and is removable from the camera lumen. The camera is used to monitor the internal membranes of the patient during the medical procedure.
According to the invention, there is provided a medical device for insertion into a cavity of a patient comprising:
an endotracheal tube for use in intubating a patient comprising a wall having a proximal end and a distal end, and a passageway extending between said proximal and distal ends, wherein after the patient is intubated, the endotracheal tube provides continuous visualization; a connector at said proximal end of said wall for connecting said endotracheal tube to a ventilating machine; an inflatable cuff attached proximate to the distal end of said wall, and a mark provided on said wall proximal of said inflatable cuff;
a camera lumen which is formed by a flexible plastic tube having a sealed window at one end thereof, said camera lumen attached to said wall of said endotracheal tube, and said sealed window being proximate to said distal end of said wall; and a separate camera insertable into said camera lumen, said camera being removable from said camera lumen.

### BRIEF DESCRIPTION OF THE DRAWINGS

The organization and manner of the structure and operation of the invention, together with further objects and advantages thereof, may best be understood by reference to the following description, taken in connection with the accompanying drawings, wherein like reference numerals identify like elements in which:
FIG. 1 is a perspective view of a medical device which incorporates the features of the present invention inserted into a patient;
FIG. 2 is a side elevation view of the medical device of FIG. 1;
FIG. 3 is an end elevation view of the medical device of FIG. 1;
FIG. 4 is alternate perspective view of the medical device of FIG. 1;
FIGS. 5A-5E are side elevation views of the medical device of FIG. 1 being inserted into a patient, and being used with a stylet and an endotracheal tube;
FIGS. 6 and 7 are perspective views of an alternate medical device which incorporates the features of the present invention;
FIG. 8 is a side elevation view of the medical device of FIGS. 6 and 7;
FIGS. 9 and 10 are perspective views of the medical device of FIGS. 6-8, with an endotracheal tube being shown for use therewith;
FIGS. 11A and 11B are side elevation views of the medical device of FIGS. 6-8 being inserted into a patient, and being used with an endotracheal tube;
FIG. 12 is a perspective view of another medical device which incorporates the features of the present invention;
FIG. 13 is a perspective view of yet another medical device which incorporates the features of the present invention;
FIG. 14 is a perspective view of the medical device of FIG. 13 in an alternate position;
FIG. 15 is a cross-sectional view of the medical device of FIG. 13;
FIG. 16 is a perspective view of another medical device which incorporates the features of the present invention;
FIGS. 17A and 17B show the medical device of FIG. 16 in use in a patient;
FIGS. 18-19C show perspective views of a further medical device which incorporates the features of the present invention;
FIG. 20 shows a perspective view of yet another medical device which incorporates the features of the present invention;
FIG. 21 shows a perspective view of yet another medical device which incorporates the features of the present invention;
FIG. 22 shows a perspective view of another medical device which incorporates the features of the present invention;
FIGS. 23 and 24 show perspective views of yet a further medical device which incorporates the features of the present invention;
FIG. 25 shows a perspective view of another medical device which incorporates the features of the present invention; and
FIG. 26 shows a perspective view of a tool which can be used with the medical device of FIG. 25; and
FIG. 27 is a schematic of a control system for use with the medical devices shown in the drawings.

### DETAILED DESCRIPTION OF THE ILLUSTRATED EMBODIMENTS

While the invention may be susceptible to embodiment in different forms, there is shown in the drawings, and herein will be described in detail, a specific embodiment with the understanding that the present disclosure is to be considered an exemplification of the principles of the invention, and is not intended to limit the invention to that as illustrated and described herein.

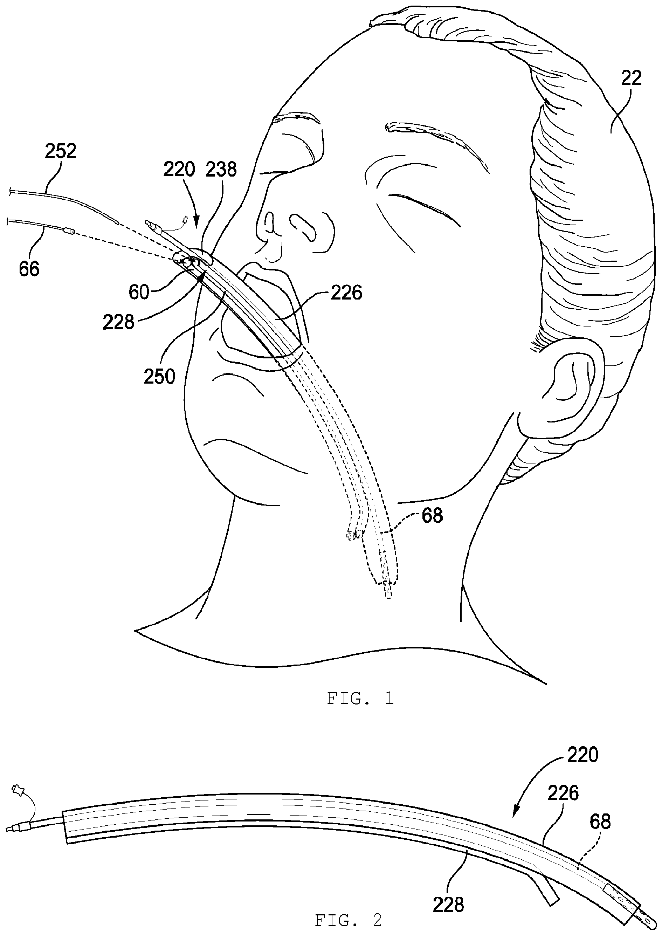

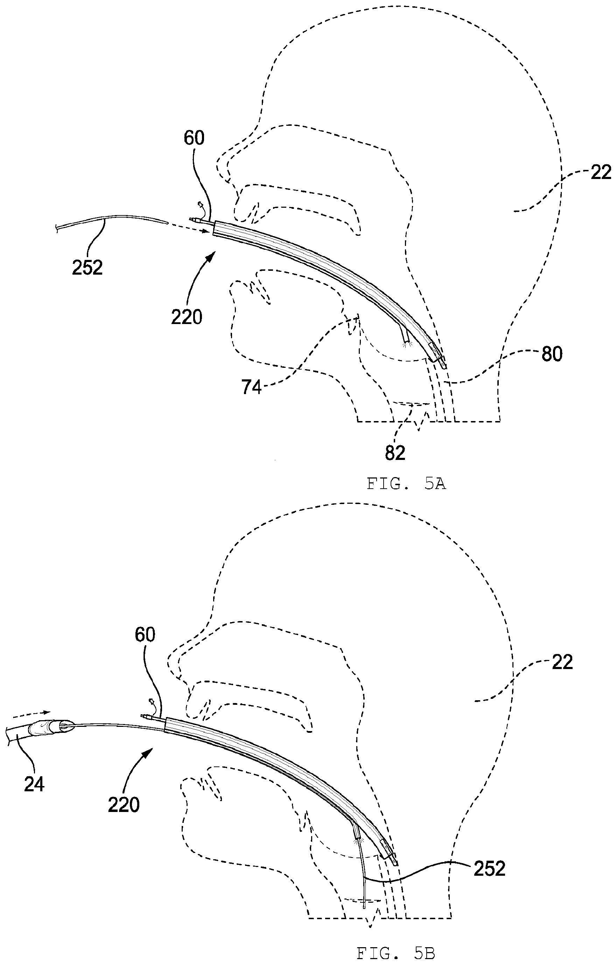

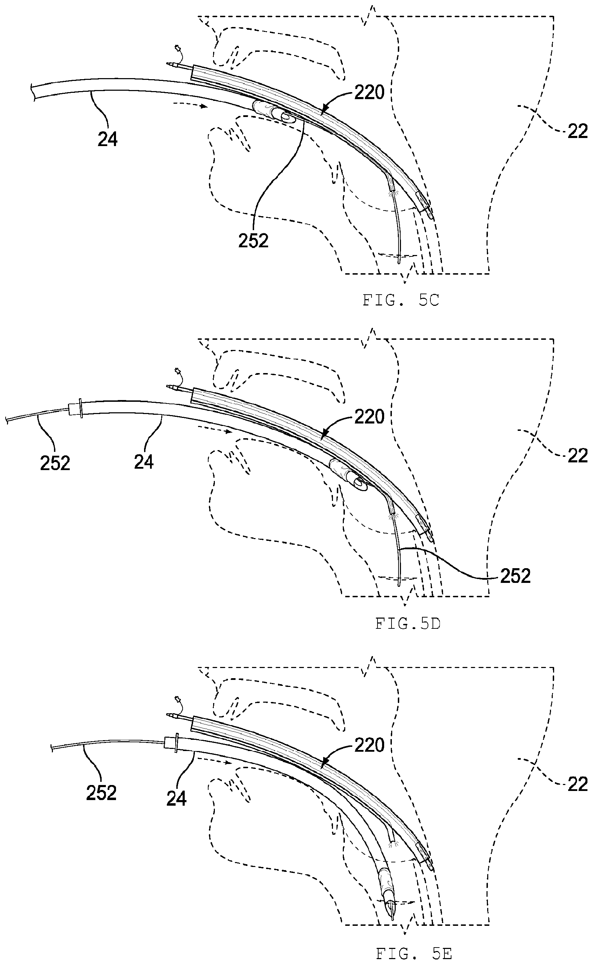

FIGS. 1-5E show a medical device 220 which is inserted into the throat of a patient 22 to determine the status of the internal membranes of the patient 22 and to provide a means for intubating the patient 22 with an endotracheal tube 24. The medical device 220 includes an esophageal tube 226 and an intubating tube 228 which are connected together. The esophageal tube 226 is used to monitor breath sounds of the patient 22, and the intubating tube 228 is used for intubation of the patient 22 using the endotracheal tube 24 as described herein.

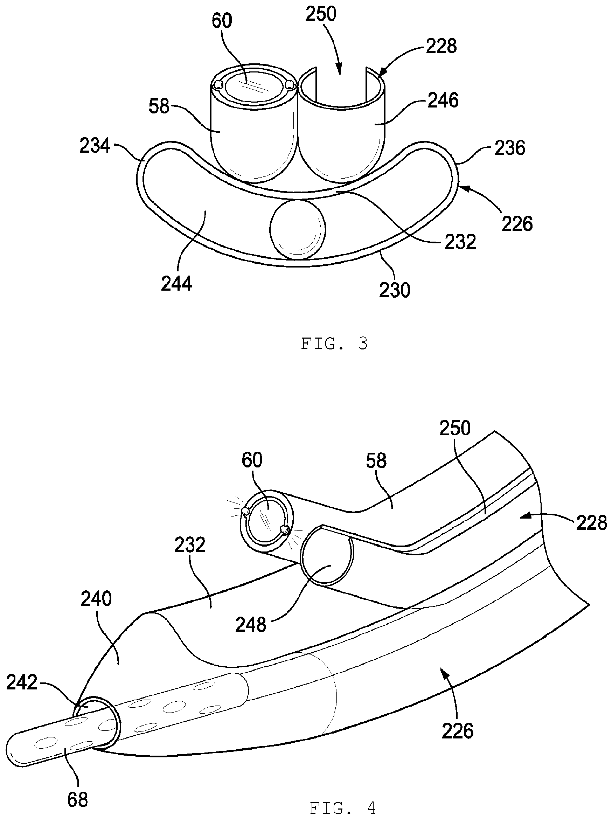

The esophageal tube 226 is formed from an elongated tube wall which as shown in FIG. 3 has first and second portions 230, 232 which are arcuate and which are connected to each other by curved end portions 234, 236. The first and second portions 230, 232 are preferably separate from each other by a distance of mm and the end portions 234, 236 are preferably separated from each other by a distance of 5 mm, however, the esophageal tube 226 may be bigger or smaller. The proximal end of the esophageal tube 226 is open and provides a proximal inlet opening 238. A generally conical end portion 240, see FIG. 4, having an aperture 242 therethrough is provided at the distal end of the tube wall. A central passageway 244 extends through the tube wall and through the conical end portion 240. The aperture 242 is in communication with the central passageway 244. The esophageal tube 226 is curved along its length. The esophageal tube 226 is formed of a relatively stiff but compliant plastics material and is preferably formed by extrusion.

A transmission lumen 68 for transmitting breath and heartbeat sounds from the patient 22 to the medical professional is provided. The transmission lumen 68 is formed by a small-diameter flexible plastic tube having a central passageway and a series of perforations at its distal end. The perforations are covered by a thin gauge plastic cap. The transmission lumen 68 seats within the central passageway 244 of the esophageal tube 226 and extends from the aperture 242. The transmission lumen 68 may seat freely in the esophageal tube 226, may be attached to the esophageal tube 226 by a friction fit with the aperture 242, or the transmission lumen 68 can be otherwise affixed to the esophageal tube 226.

The intubating tube 228 is formed from a small diameter cylindrical wall 246 having a proximal open inlet (at the end closest to the medical professional), an opposite distal open outlet (at the end furthest away from the medical professional during use of the medical device 220), a central passageway 248 extending therethrough, and a slot 250 which is in fluid communication with the central passageway 248 and extends from the proximal end (at inlet) to the distal end (at outlet) of the intubating tube 228. The intubating tube 228 is curved along its length. The intubating tube 228 has a diameter which is preferably 4 mm, however, the intubating tube 228 may be bigger or smaller. The intubating tube 228 is formed of a relatively stiff but compliant plastics material and is preferably formed by extrusion.

A camera lumen 58 which is formed by a small diameter flexible plastic tube is provided. The camera lumen 58 has a proximal end and an opposite distal end and a central passageway therethrough. The distal end of the camera lumen 58 has a clear window 60 which is sealed to the camera lumen 58 to close the end of the central passageway. As a result, the camera lumen 58/window 60 are impervious to gases/fluids such that entry of fluids and other matter into the camera lumen 58 is prevented. A pair of LED lights may be formed in the wall of the camera lumen 58 on opposite sides of the window 60. If LED lights are provided in the camera lumen, wires are molded into the camera lumen 58 and extend from the proximal end thereof for connection to a suitable power source. A non-disposable camera 66 can be easily slid into and removed from the sealed camera lumen 58/window 60 combination. Instead of providing separate LED lights in the camera lumen 58, the camera 66 and LED lights (or other source of lighting, including a camera with its own built-in lighting) can be incorporated into a single non-disposable device which is insertable and removeable from the camera lumen 58.

The intubating tube 228 is affixed, such as by ultrasonic welding, to the esophageal tube 226 along the second wall portion 232. The camera lumen 58 is affixed, such as by ultrasonic welding, to the esophageal tube 226 along the second wall portion 232 and is proximate to the intubating tube 228. The camera 66 and LED lights (or other source of lighting, including a camera with its own built-in lighting) can be incorporated into a single device which is insertable and removeable from the camera lumen 58. The camera 66 is situated to provide the best angle for viewing the tissues of the patient 22 when the medical device 220 is being inserted into the throat of a patient 22. As a result, the intubating tube 228 and the camera lumen 58 are situated side-by-side. The inlets of the esophageal tube 226, the intubating tube 228 and the camera lumen 58 generally align with each other. The outlets do not align with each other; instead, the outlets of the intubating tube 228 and the camera lumen 58 are spaced proximally a predetermined distance from the outlet of the esophageal tube 226. The slot 250 in the intubating tube 228 is opposite to the point of weldment of the intubating tube 228 to the esophageal tube 226 to provide a means for insertion of the endotracheal tube 24 into the patient 22 as described herein.

In use, the medical professional inserts the medical device 220 through the mouth and into the throat of the patient 22. The esophageal tube 226 slides against the hard palate and then against the soft palate and into the pharynx of the patient 22. The medical device 220 will flex to assume a curved shape to conform to the throat of the patient 22. The medical device 220 does not block the airway of the patient 22 so the patient 22 can breathe on his/her own. The generally conical end wall 240 of the esophageal tube 226 enters into the upper end of esophagus 80 such that the transmission lumen 68 is positioned within the esophagus 80 and is located closest to the lungs of the patient 22. The distal outlet of the intubating tube 228 is open to the glottis of the patient 22. During this entire procedure of insertion, the camera 66 provides constant visualization of the tissues during insertion of the medical device 220 into the patient 22. Because the camera 66 provides constant visualization of the tissues during insertion of the medical device 220 into the patient, the medical professional can be assured that the medical device 220 is being properly inserted and positioned in the throat of the patient 22 with limited trauma to the patient 22. Since the camera lumen 58 terminates proximally of the esophageal tube 226 and does not enter into the esophagus 80, the medical professional can see the vocal folds 82 via the camera 66. Since the camera 66 is constantly operating during insertion and through the entire medical procedure, the medical professional can constantly visually confirm that the patient 22 is breathing by the rhythmic opening and closing of the vocal folds 82. The constant visualization of the laryngeal inlet and the vocal folds 82 of the patient 22 can make earlier diagnoses of issues, for example, but not limited to, secretions, tumors, paralyzed vocal folds, apnea, bleeding, and abnormal anatomy, as well as other potentially harmful effects to the patient 22.

The medical device 220 is used to insert the endotracheal tube 24. First, a stylet 252, which is known in the art, is inserted into the intubating tube 228 and the distal end thereof preferably extends past the distal end of the intubating tube. The stylet 252 rides along the portion of the wall 246 of the intubating tube 228 which is proximate to the point of weldment to the esophageal tube 226, and, as such, will not be prone to falling through the slot 250. Thereafter, the distal end of the endotracheal tube 24 is threaded over the proximal end of the stylet 252 (the portion of the stylet 252 which extends from the proximal end of the intubating tube 228). As the endotracheal tube 24 is pushed along the stylet 252, the stylet 252 is pulled upwardly to release it from the intubating tube 228 by the stylet 252 moving through the slot 250. As a result, the endotracheal tube 24 is guided along the proper path by the stylet 252 and the intubating tube 228. Once the stylet 252 is completed released from the intubating tube 228, the endotracheal tube 24 can be further inserted through the vocal folds 82 of the patient 22 under the visualization provided by the camera 66. The medical professional can thus guide the endotracheal tube 24 through the vocal folds 82 and into the trachea, and inflate the cuff of the endotracheal tube 24, under the constant visualization provided by the camera 66. At times, the medical device 220 may be advanced, pulled back, or turned from side to side, to maintain the proper trajectory of the endotracheal tube 24 through the vocal folds 82. This is easily accomplished since there is constant visualization of the tissues via the camera 66.

When the medical device 220 is seated in the throat of the patient 22, the distal end of the transmission lumen 68 is positioned within the esophagus 80 which enables breath and heartbeat sounds to be easily transmitted through the transmission lumen 68 to the medical professional monitoring the patient 22 as described herein.

As a result of the structure of the medical device 220, the intubating tube 228 is located away from the epiglottis 74 of the patient 22 when the medical device 220 is positioned within the patient 22. This minimizes the ability of the epiglottis 74 to block the insertion of the endotracheal tube 24.

Because of the structure of the medical device 220, the patient 22 does not have to be laying on his/her back to effect intubation. The patient 22 can be sitting in a chair, or lying face down.

The video information from the camera 66 and the information from the transmission lumen 68 are transmitted to a microprocessor 82 via appropriate means, such as wires, wireless, Bluetooth, etc., which in turn can transmit the information to another computer, mobile devices, a mobile station and the like, via appropriate means, such as wires, wireless, Bluetooth, etc., and then this information can be accessed by appropriate personnel. This microprocessor 82 can be on-site where the procedure is being performed or can be remote from the procedure site. For example, the information can be supplied to the nurses' station and the nurse on duty will be able to instantly know if the patient 22 is breathing by the visual confirmation that the vocal folds are opening and closing and by hearing breath and heart sounds. The medical professional will be able to interpret the depth of anesthesia by looking at the rhythmic movement of the vocal folds as well as other diagnoses previously mentioned. Other medical personnel can be hundreds of miles away and still be able to monitor, advise, confirm, and diagnose without the patient 22 being in close physical proximity to that medical personnel. Since the camera 66 is constantly operating, medical personnel can tell at any time if the patient 22 is properly ventilated/intubated and is breathing.

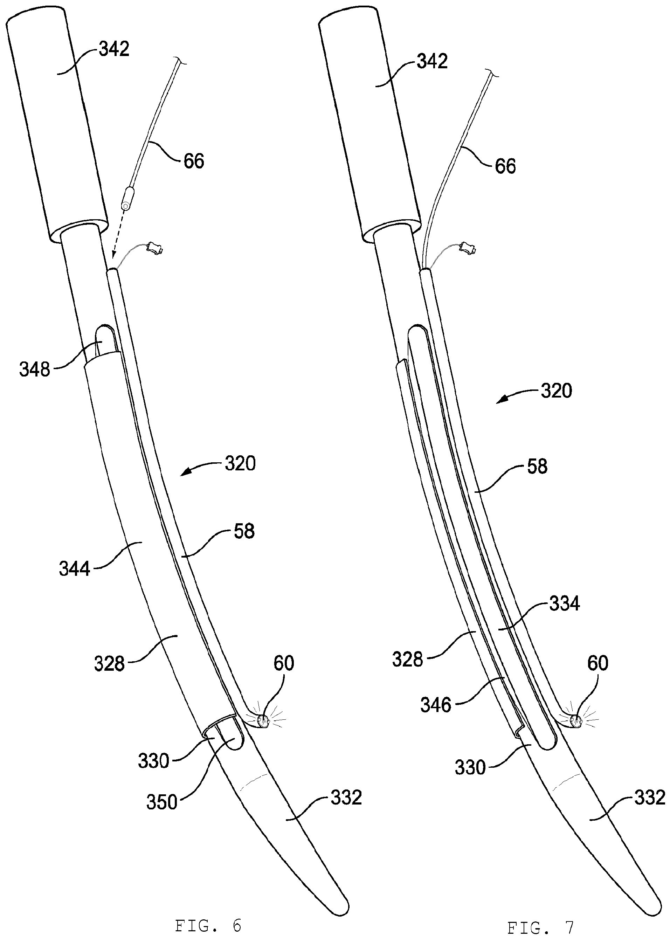

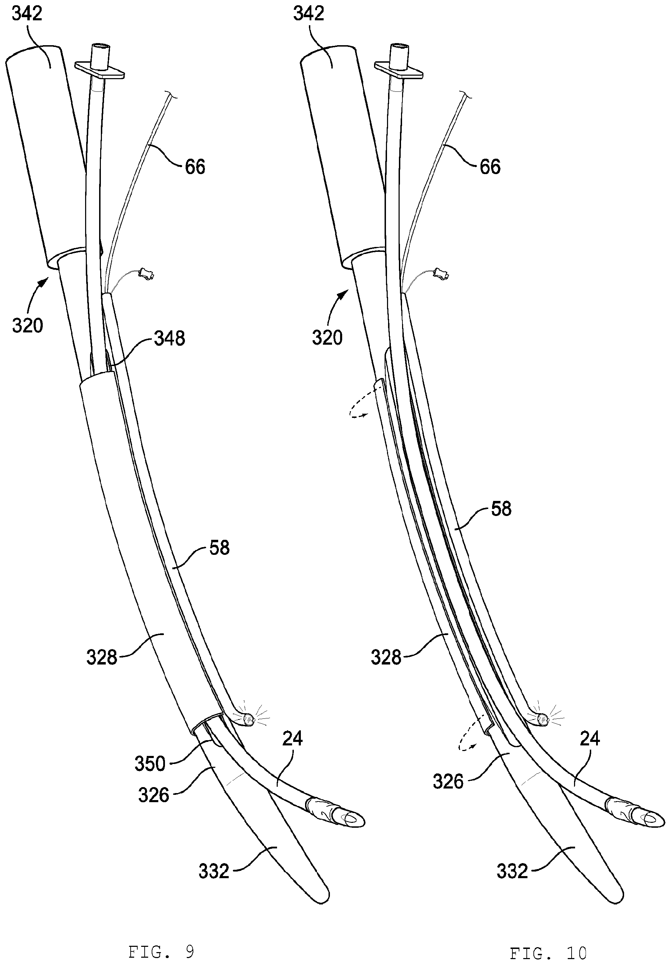

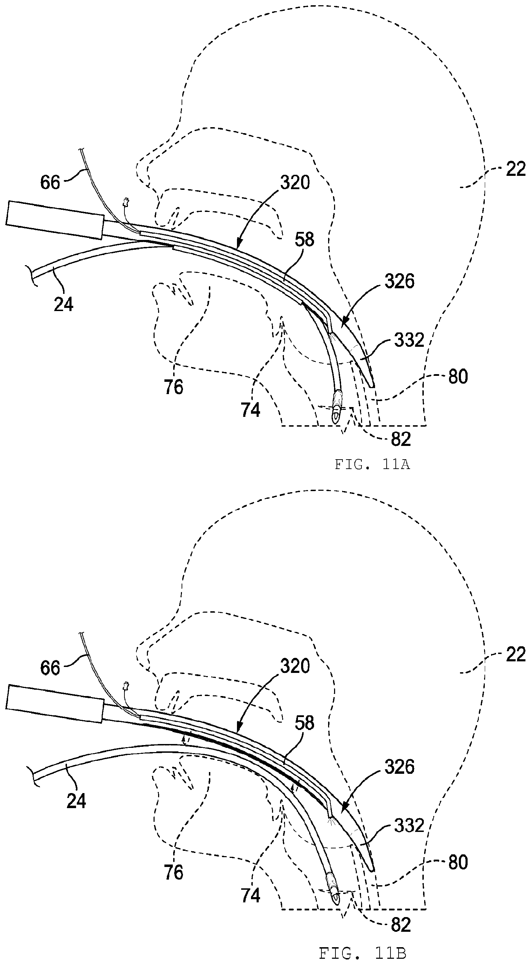

FIGS. 6-11B show an alternate medical device 320 which is inserted into the throat of a patient 22 to determine the status of the internal membranes of the patient 22 and to provide a means for intubating the patient 22 with an endotracheal tube 24. The medical device 320 includes an intubating tube 326 and a sleeve 328 which are connected together. The sleeve 328 is rotatable relative to the intubating tube 326.

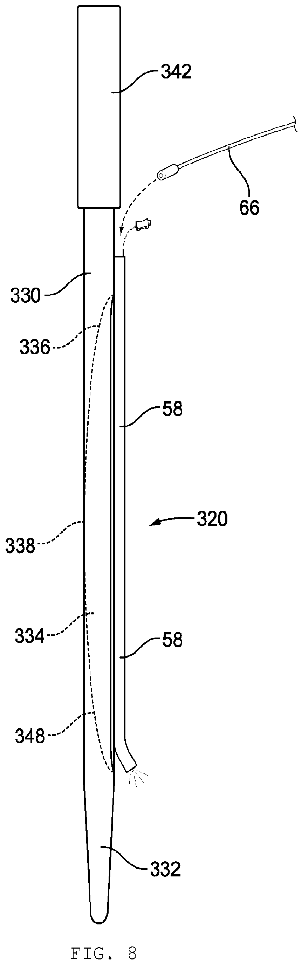

The intubating tube 328 is formed by an elongated cylindrical body 330 which has a generally conical tip 332 at its distal end. The intubating tube 228 is made of a flexible plastic material. The body 330 has an elongated recess 334 therein which generally extends from the proximal end of the body 330 to the distal end of the body 330. The recess 334 is curved along its length such that in cross-section it is generally arcuate as shown in FIG. 8. As a result, a proximal curved ramp surface 336 is formed by the recess 334 and extends from the outer surface of the body 330 proximate to the distal end thereof distally to the apex 338 of the recess 334, and a distal curved ramp surface 340 is formed by the recess 334 and extends from the outer surface of the body 330 proximate to the distal end thereof proximally to the apex 338 of the recess 334. An enlarged cylindrical handle 342 is provided at the distal end of the body 330. The tip 332 has a weight provided therein to make the tip 332 heavier than the remainder of the medical device 320. A camera lumen 58/window 60 and separate camera 66 like that of the previous embodiment of the medical device 220 are provided and the specifics are not repeated herein. The camera lumen 58 is preferably positioned beside the recess 334 and the distal end of the camera lumen 58 is proximate to the distal end of the recess 334. The camera lumen 58 is suitably attached to the intubating tube 326 by suitable means, such as ultrasonic welding. Alternatively, the camera lumen 58 can be provided integral with the intubating tube 326.

The sleeve 328 is formed from generally C-shaped wall 344 which defines a slot 346 between the opposite ends of the C-shaped wall 344. The sleeve 328 is made of a flexible plastic material. The sleeve 328 has a length which is less than the length of the recess 334, surrounds a portion of the body 330, and is rotatable relative the body 330 to cover and uncover the majority of the recess 334.

In use, the sleeve 328 is rotated relative to the body 330 such that the recess 334 is partially blocked. The proximal and distal ends of the recess 334 are not blocked by the sleeve 328 such that proximal and distal openings 348, 350 are formed as shown in FIG. 6. The patient 22 then swallows the medical device 320, or the medical professional inserts the medical device 320 through the mouth and into the throat of the patient 22. During this insertion, the recess 334 is proximate to the tongue 76 of the patient 22. The intubating tube 326 slides against the hard palate and then against the soft palate and into the pharynx of the patient 22. The medical device 320 will flex to assume a curved shape to conform to the throat of the patient 22. The medical device 320 does not block the airway of the patient 22 so the patient 22 can breathe on his/her own. The generally conical tip 332 of the intubating tube enters into the upper end of esophagus 80. The proximal opening 348 is positioned exterior to the mouth of the patient 22. The distal opening 350 is open to the glottis of the patient 22. During this entire procedure of insertion, the camera 66 provides constant visualization of the tissues of the patient 22. Because the camera 66 provides constant visualization, the medical professional can be assured that the medical device 320 is being properly inserted and positioned in the throat of the patient 22 with limited trauma to the patient 22. Since the camera lumen 58 terminates proximally of the intubating tube 326 and does not enter into the esophagus 80, the medical professional can see the vocal folds 82 via the camera 66. Since the camera 66 is constantly operating during insertion and through the entire medical procedure, the medical professional can constantly visually confirm that the patient 22 is breathing. The constant visualization of the laryngeal inlet and the vocal folds 82 of the patient 22 can make earlier diagnoses of issues, for example, but not limited to, secretions, tumors, paralyzed vocal folds, apnea, bleeding, and abnormal anatomy, as well as other potentially harmful effects to the patient 22.

The medical device 320 is then used to insert the endotracheal tube 24. The distal end of the endotracheal tube 24 is inserted into the proximal opening 348 and pushed along the length of the recess 334 until the distal end and cuff of the endotracheal tube 24 exit through the distal opening 350 and passes through the vocal folds of the patient 22. The ramp surface 340 at the distal end of the recess 334 aids in properly positioning the endotracheal tube 24 relative to the glottis of the patient 22. The camera 66 is used to determine the positioning of the endotracheal tube 24 and the medical professional can adjust the position of the medical device 320 using this constant visualization provided by the camera 66 to ensure proper entry of the endotracheal tube 24 through the vocal folds 82 and into the trachea of the patient 22. At times, the medical device 320 may be advanced, pulled back, or turned from side to side, to maintain the proper trajectory of the endotracheal tube 24 through the vocal folds 82. This is easily accomplished since there is constant visualization of the tissues via the camera 66. Once the endotracheal tube 24 is properly positioned, the sleeve 328 is rotated relative to the intubating tube 326 and the endotracheal tube 24 is released from the intubating tube 326. The medical device 320 can then be removed from the patient 22 if desired.

A transmission lumen (not shown) like that of the previous medical device 220 may be provided and attached to the intubating tube 326. If provided, when the medical device 320 is seated in the throat of the patient 22, the distal end of the transmission lumen is positioned within the esophagus 80 which enables breath and heartbeat sounds to be easily transmitted along the length of the transmission lumen to the medical professional monitoring the patient 22 as described herein.

As a result of the structure of the medical device 320, the intubating tube 326 is located away from the epiglottis 74 of the patient 22 when the medical device 320 is positioned within the patient 22. This minimizes the ability of the epiglottis 74 to block the insertion of the endotracheal tube 24.

Because of the structure of the medical device 320, the patient 22 does not have to be laying on his/her back to effect intubation. The patient 22 can be sitting in a chair, or lying face down. The medical devices 220, 320 provide a new methodology of intubating the patient 22 by placing the medical device 220, 320 into the esophagus 80 of the patient 22 and working upwardly toward the trachea.

Like that of the previous medical device 220, the video information from the camera 66 and the information from the transmission lumen are transmitted to a microprocessor 82 via appropriate means, such as wires, wireless, Bluetooth, etc., which in turn can transmit the information to another computer, mobile devices, a mobile station and the like, via appropriate means, such as wires, wireless, Bluetooth, etc., and then this information can be accessed by appropriate personnel. This microprocessor 82 can be on-site where the procedure is being performed or can be remote from the procedure site. For example, the information can be supplied to the nurses' station and the nurse on duty will be able to instantly know if the patient 22 is breathing by the visual confirmation that the vocal folds are opening and closing and by hearing breath and heart sounds. The medical professional will be able to interpret the depth of anesthesia by looking at the rhythmic movement of the vocal folds 82 as well as other diagnoses previously mentioned. Other medical personnel can be hundreds of miles away and still be able to monitor, advise, confirm, and diagnose without the patient 22 being in close physical proximity to that medical personnel. Since the camera 66 is constantly operating, medical personnel can tell at any time if the patient 22 is properly ventilated/intubated and is breathing.

As an alternative, the endotracheal tube 24 can be seated within the recess 334 prior to insertion of the medical device 320 into the patient 22. The distal portion of the endotracheal tube 24 is preferably trapped between the sleeve 328 and the body 330 when the medical device 320 is first inserted to prevent damage to the cuff of the endotracheal tube 24.

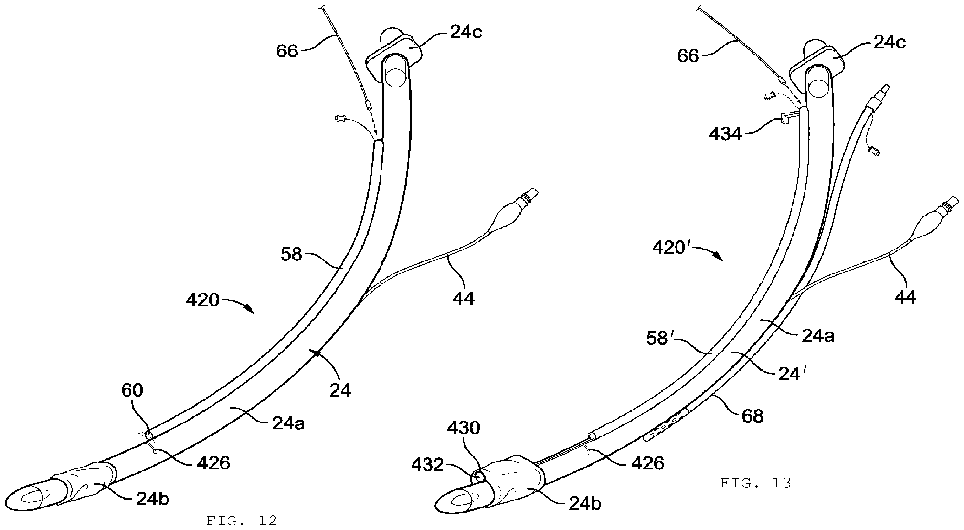

FIG. 12 shows a medical device 420 which includes an endotracheal tube 24 having the camera lumen 58/window 60 affixed thereto. The camera lumen 58/window 60 and separate camera 66 like that of the previous embodiments of the devices 220, 320 are provided and the specifics are not repeated herein.

As is known in the art, the endotracheal tube 24 includes a cylindrical wall 24a formed of a relatively stiff but compliant plastics material and is preferably formed by extrusion which has a central passageway therethrough, a cuff 24b attached proximate to the distal end of the wall 24a which is inflatable via inflation line 44 which is formed by a small-diameter flexible plastic tube. The proximal end of the tube 24a has a connector 24c for connecting the endotracheal tube 24 to a ventilating machine in a known manner. The prior art endotracheal tube 24 is modified in that a mark 426 is provided thereon which is preferably one inch proximal of the cuff 24b.

The camera lumen 58 extends along the length of the wall 24a and the distal end of the camera lumen 58 is proximate to the mark 426. The camera lumen 58 is suitably attached to the wall 24a by suitable means, such as ultrasonic welding. Alternatively, the camera lumen 58 can be provided integral with the wall 24a.

In use, the medical professional inserts the medical device 420 through the mouth and into the throat of the patient 22. The endotracheal tube 24 then passes through the vocal folds and into the trachea of the patient 22. The medical device 420 will flex to assume a curved shape to conform to the throat of the patient 22. During this entire procedure of insertion, the camera 66 provides constant visualization of the tissues during insertion of the medical device 420 into the patient 22. Because the camera 66 provides constant visualization of the tissues during insertion of the medical device 420 into the patient, the medical professional can be assured that the medical device 420 is being properly inserted and positioned in the throat of the patient 22 with limited trauma to the patient 22. The medical professional can adjust the position of the medical device 420 using this constant visualization provided by the camera 66 to ensure proper entry of the endotracheal tube 24 through the vocal folds and into the trachea of the patient 22. At times, the medical device 420 may be advanced, pulled back, or turned from side to side, to maintain the proper trajectory of the endotracheal tube 24 through the vocal folds. This is easily accomplished since there is constant visualization of the tissues via the camera 66.

The portion of the endotracheal tube 24 distal to the mark 426 passes through the vocal folds, however, the camera lumen 58 does not pass through the vocal folds. As such, the camera 66 is used to continuously visualize the vocal folds and to view the portion of the endotracheal tube 24 which is distal to the vocal folds (when the vocal folds are open) to determine if the endotracheal tube 24 has been properly positioned. Since camera lumen 58 does not pass through the vocal folds, this provides a smaller dimension of material passing through the vocal folds. Since the camera 66 is constantly operating during insertion and through the entire medical procedure, the medical professional can constantly visually confirm that the patient 22 is breathing. The constant visualization of the laryngeal inlet and the vocal folds of the patient 22 can make earlier diagnoses of issues, for example, but not limited to, secretions, tumors, paralyzed vocal folds, apnea, bleeding, and abnormal anatomy, as well as other potentially harmful effects to the patient 22.

A transmission lumen (not shown) like that of the previous medical devices 220, 320 may be provided and attached to the endotracheal tube 24. If provided, when the medical device 420 is seated in the throat of the patient 22, the distal end of the transmission lumen is positioned proximate to the esophagus which enables breath and heartbeat sounds to be easily transmitted through the transmission lumen to the medical professional monitoring the patient 22.

FIGS. 13-15 shown a modified medical device 420' to that shown in FIG. 12 which includes a modified endotracheal tube 24' and a modified camera lumen 58'. The camera lumen 58' is attached to the wall 24a by means which allow the camera lumen 58 to slide relative to the wall 24a. As shown, a dovetail joint 428, formed of a tongue on one of the camera lumen 58 and the wall 24a and of a groove on the other of the camera lumen 58 and the wall 24a, mounts the camera lumen 58 to the wall 24a. A small cylindrical tube 430 is affixed to the wall 24a by suitable means, such as ultrasonic welding, and is provided between the cuff 24b and the wall 24a. A passageway 432 is provided by the small cylindrical tube 430. The distal end of the tube 430 is sealed with a window. The small cylindrical tube 430 is positioned at the end of the structure forming the portion of the dovetail joint 428 on the wall 24b. The camera lumen 58' has a handle 434 attached to its distal end to allow the medial professional to grasp the camera lumen 58' to manipulate the position of the distal end of the camera lumen 58' as described herein.

In use, the medical professional can grasp the handle 434 to slide the camera lumen 58, with the camera 60 mounted therein, along the wall 24a via the inter-engagement of dovetail joint 428 and to slide the distal end of the camera lumen 58/camera 66 through the small cylindrical tube 430. This allows the medical professional to continuously view the tissues of the patient on either side of the vocal folds of the patient 22.

A transmission lumen 68 is affixed to the endotracheal tube 24', preferably to the wall 24a opposite to that where the camera lumen 58' is located. When the medical device 420 is seated in the throat of the patient 22, the distal end of the transmission lumen 68 is positioned proximate to the esophagus which enables breath and heartbeat sounds to be easily transmitted through the transmission lumen 68 to the medical professional monitoring the patient 22.

In the embodiments of the medical device 420, 420', the camera lumen 58, 58'/window 60 could instead be mounted within the endotracheal tube 24, 24', with the mark 426 provided on an interior surface of the endotracheal tube 24, 24'.

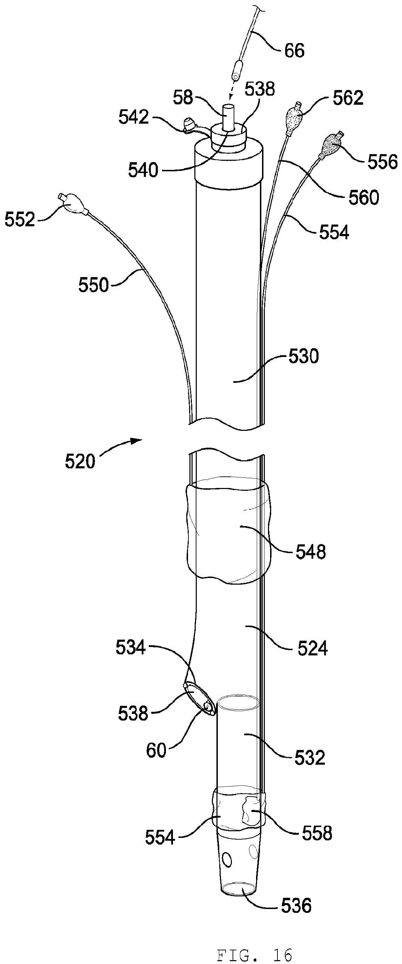

FIGS. 16-17B show a medical device 520 formed of a dual branch endotracheal tube 524 which incorporates the sealed camera lumen 58/window 60 and separate camera 66 which can be inserted therein and removed therefrom as described herein. The camera lumen 58/window 60 and separate camera 66 are like that of the previous embodiments of the medical devices 220, 320, 420, 420' and the specifics are not repeated herein. The dual branch endotracheal tube 524 is used to separate the left and right bronchus 526, 528 from each other for surgical purposes and can be used as a normal endotracheal tube which is normally seated in the trachea of the patient 22. In the prior art, separation of the left and right bronchus 526, 528 from each other is usually accomplished using two lumens which tends to be cumbersome.

The endotracheal tube 524 is formed from a main cylindrical wall 530 having a proximal end (end closest to the medical professional during use) and a distal end (end furthest from the medical professional during use), a first branch cylindrical wall 532 extending from the distal end of the main wall 530, and second branch cylindrical 534 wall extending from the distal end of the main wall 530. The first and second branch walls 532, 534 are smaller in dimension than the main wall 530, but when the dimensions of the branch walls 532, 534 are combined, this dimension is approximately equal to the dimension of the main wall 530. As a result, the endotracheal tube 524 has a single inlet port at its proximal end and first and second outlet ports at its distal end. A central passageway extends through the main wall 530, and branch passageways extend through the branch walls 532, 534, each of which are in communication with the central passageway through the main wall 530. The distal end of the first branch wall 532 has a first port 536 which terminates proximally of the distal end of the second branch wall 534 which forms a second port 538. The second port 538 is angled relative to the first port 536.

The proximal end of the endotracheal tube 524 is closed with a cap 538. The cap 538 has a first aperture 540 therethrough which can be closed with a plug 542 in a known manner, and a second aperture 544 provided through an extension 544 which extends perpendicular to the centerline of the main wall 530. A ventilator is attached to the extension 544 in a known manner to provide positive air pressure to the medical device 520.

An inflatable cuff 548 surrounds the main wall 530 at a position which is spaced from the branch walls 532, 534. An inflation line 550 and its associated pilot 552 is attached to this inflatable cuff 548. An inflatable cuff 554 surrounds the first branch wall 532 at a position which is spaced from the distal port 536. An inflation line 554 and its associated pilot 556 is attached to this inflatable cuff 554. An inflatable cuff 558 (shown in full line in the figures for ease in understanding) is positioned interiorly within the first branch wall 532 at a position which is spaced from the distal port 536. An inflation line 560 and its associated pilot 562 is attached to this interior inflatable cuff 558. The camera lumen 58/window 60 extends through the aperture 540 in the cap 538, through the central passageway in the main wall 530 and into the second branch passageway through the second wall 534 and preferably terminates at, or proximate to, the second port 538. The cap 538 and the lumen 58 can form a frictional fit to prevent the camera lumen 58 from 28 disengaging from the cap 538. The camera 66 is then is inserted into the camera lumen 58.

The medical professional inserts the medical device 520, with all of the cuffs 548, 554, 558 in the deflated condition, through the mouth and into the throat of the patient 22. The camera 66 mounted in the medical device 520 provides constant visualization of the tissues of the patient 22 during this insertion.

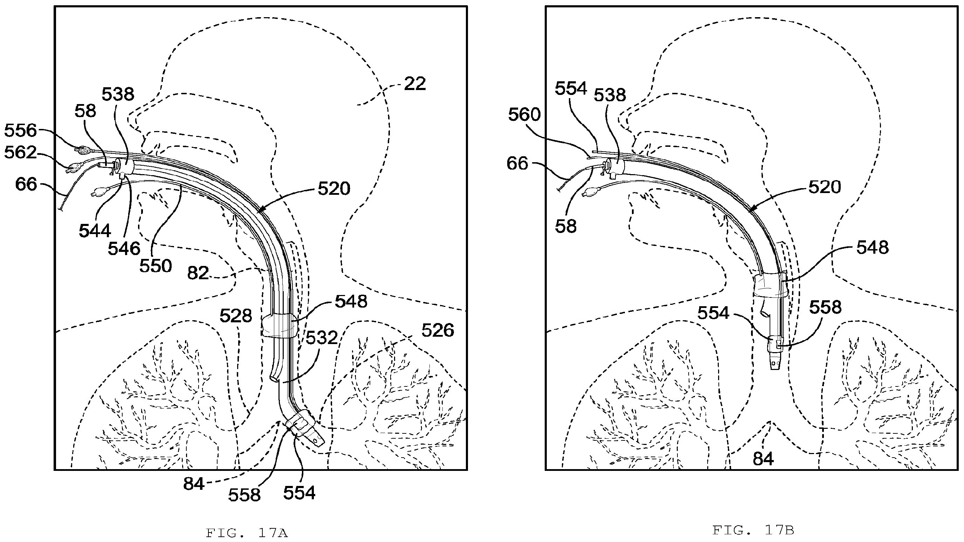

The medical device 520 can be placed in the patient 22 in one of the three positions. In a first position, the medical device 520 can be placed such that the main wall 530 and the second branch wall 534 are in the trachea of the patient 22 and past the vocal folds 82, and the first branch wall 532 is in the left main stem bronchus 526 of the patient 22, as shown in FIG. 17A. In a second position, the medical device 520 can be placed such that the main wall 530 and the second branch wall 534 are in the trachea of the patient 22 and past the vocal folds 82, and the first branch wall 532 is in the right main stem bronchus of the patient 22 (not shown). In a third position, the medical device 520 can be placed such that the main wall 530 and both branch walls 532, 534 are in the trachea of the patient 22, past the vocal folds 82, but not past the carina 84, as shown in FIG. 17B.

In use, the medical professional inserts the medical device 520 into the patient 22. The camera 66 is used to continuously visualize the position of the medical device 520 during this insertion. For surgery, the medical device 520 is positioned such that the main wall 530 and the second branch wall 534 seat in the trachea of the patient 22 and the first branch wall 532 seats into one of the bronchus of the patient 22 (left as shown in the drawings). Since the camera 66 is positioned in the trachea of the patient 22, the carina 84 of the patient 22 is always within view and the medical professional will know that the first branch wall 532 is positioned within the bronchus of the patient 22. Once properly positioned, the external cuffs 548, 554 are inflated to hold the medical device 520 in place. Cuff 548 engages with the trachea of the patient 22, and cuff 554 engages with the bronchus of the patient 22. The camera 66 provides visual confirmation that the cuff 554 has been inflated in the bronchus. When the surgeon wants to operate on the lung, the medical professional disconnects the ventilator from extension 544 to allow both lungs to deflate. Thereafter, the internal cuff 558 is inflated to block airflow through the branch wall to the bronchus in which the first branch wall 532 is seated. Thereafter, ventilation is resumed such that the lung which will not be operated upon is functioning. Once the medical procedure is completed, all of the cuffs 548, 554, 558 are deflated and the medical device 520 is pulled proximally to remove the second branch wall 532 from the bronchus. As a result, the main wall 530 and both branch walls 532, 534 are seated within the trachea. The camera 66 again provides visual confirmation of the position of the medical device 520 in the trachea as the carina 84 can be seen.

Once the medical device 520 is positioned completely in the trachea, the pilots 556, 560 to the inflation lines 554, 560 to the distal cuffs 554, 558 are cut as shown in FIG. 17B to prevent their inflation. The main cuff 548 is then re-inflated and the medical device 520 functions as a normal endotracheal tube. This medical device 520 can be modified to include the camera lumen shown in FIGS. 12-15 to allow the medical professional to view the tissues above or below the vocal folds 82.

This structure presents a distinct advantage over prior art double lumen endotracheal tubes. Prior art double lumen endotracheal tubes are much wider than the present medical device 520, which may cause undue harm to the patient.

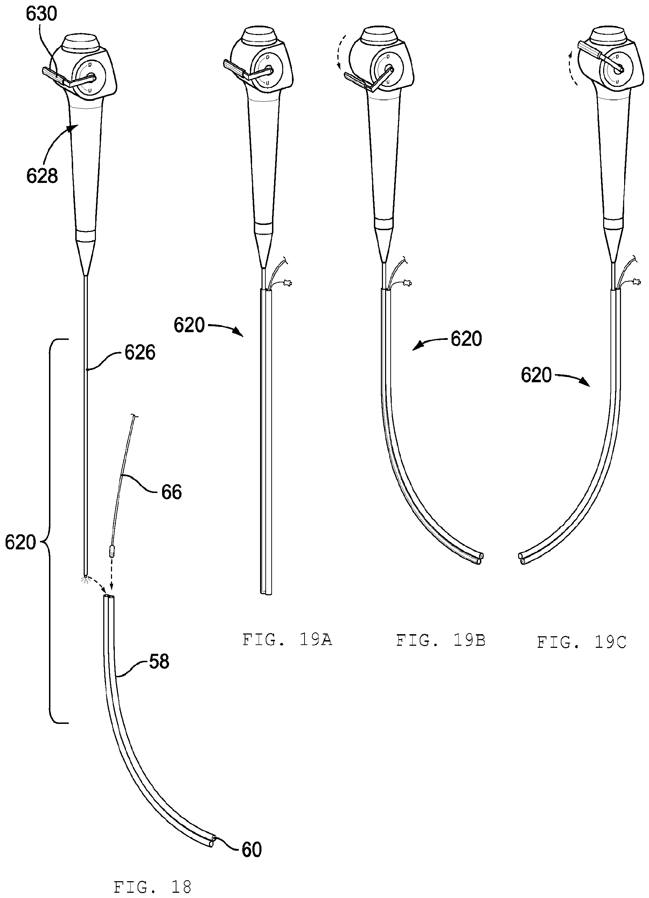

FIGS. 18-19C show a medical device 620 which is a modified bronchoscope which has the camera lumen 58/window 60 and separate camera 66. The camera lumen 58/window 60 and separate camera 66 are like that of the previous embodiments of the medical devices 220, 320 and the specifics are not repeated herein.

In the prior art, bronchoscopes use a fiber optic line, the position of which can be manipulated by the mechanism at the proximal end of the fiber optic line. As is known in the prior art, the handle on the mechanism can be toggled to cause the fiber optic line to curved either to the left or to the right. Prior art bronchoscopes are expensive because of the built-in fiber optics and if this fiber optic line is compromised, the entire bronchoscope must be replaced.

The medical device 620 replaces the fiber optic line of the prior art bronchoscope, with an elongated plastic line 626 which is flexible, yet maintains its rigidity. The position of the plastic line 626 is manipulated by the same mechanism 628 which is known in the prior art bronchoscope to cause the plastic line 628 to curve to the left or to the right (shown in FIGS. 19B and 19C) by toggling the handle 630.

The medical device 620 has the camera lumen 58/window 60 affixed to the elongated plastic line 626 by suitable means, such as ultrasonic welding, such that the plastic line 626 and the camera lumen 58 are side-by-side. The distal ends of the plastic line 626 and the camera lumen 58 preferably terminate at the same point. As is described herein with respect to the other embodiments, the camera 66 is removably placed in this sealed camera lumen 58.

In use, the medical professional inserts the plastic line 626/camera lumen 58 with the camera 66 mounted therein into the patient 22 and uses it like a prior art bronchoscope. The camera 66 provides constant visualization of the tissues of the patient 22.

The medical device 620 can be used in place of the camera lumen 58 in the medical devices 420, 420', 520 disclosed herein. The medical device 620 can be used to place the medical devices 420, 420', 520 in the patient 22.

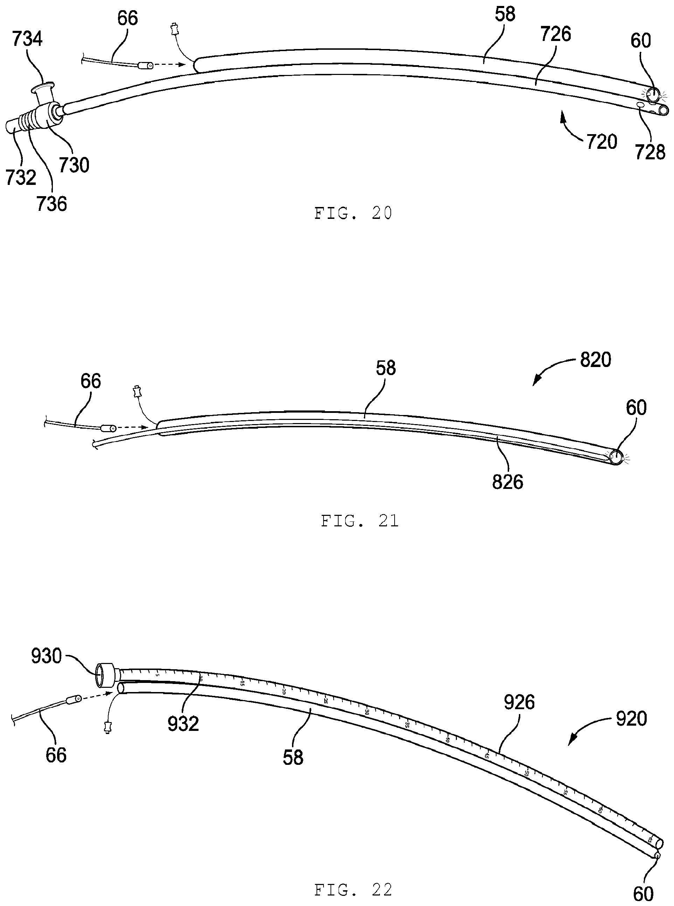

FIG. 20 shows a medical device 720 which is used to provide suction to a cavity of the patient 22, such as the lungs, the chest cavity, etc. to drain fluids/air from the cavity. The medical device 720 includes a cylindrical suction tube 726 which is known in the art which is attached to the camera lumen 58/window 60 and separate camera 66. The camera lumen 58/window 60 and separate camera 66 are like that of the previous embodiments of the medical devices 220, 320, 420, 420', 520, 620 and the specifics are not repeated herein.

The suction tube 726 has a proximal open inlet (at the end closest to the medical professional), an opposite distal outlet (at the end furthest away from the medical professional during use) and a central passageway extending therethrough. The suction tube 726 is curved along its length. The distal end of the suction tube 726 has a series of perforations 728. The suction tube 726 is formed of a relatively stiff but compliant plastics material and is preferably formed by extrusion. The proximal end of the suction tube 726 has a connector 730 attached thereto as is known in the art. The connector 730 has two ports 732, 734 which are perpendicular to each other. As is known in the art, port 732 is attached to a suction device (not shown); the ribs 736 on the port 732 help retain the suction device thereon. When the suction device is turned on, air will be entrained from the open port 734. The medical professional places his/her thumb or otherwise blocks the port 734 to cause the suction tube 726 to suck fluids from the distal end through the perforations 728.

The camera lumen 58 is affixed, such as by ultrasonic welding, to the suction tube 726, such that the suction tube 726 and the camera lumen 58 are side-by-side. The distal ends of the suction tube 726 and the camera lumen 58 preferably terminate at the same point. As is described herein with respect to the other embodiments, the camera 66 is removably placed in this sealed camera lumen 58.

In use, the medical professional inserts the medical device 720 through the endotracheal tube 24, through a drain line (not shown) or directly into the patient's cavity. The camera 66 provides constant visualization of the tissues during insertion and use of the medical device 720 into the patient 22 and the medical professional can be assured that the medical device 720 is being properly positioned in the patient 22 with limited trauma to the patient 22. The constant visualization of the tissues of the patient 22 can make earlier diagnoses of issues, for example, but not limited to, secretions, tumors, paralyzed vocal folds, apnea, bleeding, and abnormal anatomy, as well as other potentially harmful effects to the patient 22.

The medical device 720 can be used in combination with many of the other medical devices disclosed herein.

FIG. 21 shows a medical device 820 which provides a stylet 826 which is known in the art which is attached to the camera lumen 58/window 60 and separate camera 66. The camera lumen 58/window 60 and separate camera 66 are like that of the previous embodiments of the devices 220, 320, 420, 420', 520, 620, 720 and the specifics are not repeated herein.

The camera lumen 58 is attached to the stylet 826 by suitable means, such as ultrasonic welding or a dovetail joint between the camera lumen 58 and the stylet 82, such that the stylet 826 and the camera lumen 58 are side-by-side. The distal ends of the stylet 826 and the camera 32 lumen 58 preferably terminate at the same point. As is described herein with respect to the other embodiments, the camera 66 is removably placed in this sealed camera lumen 58 to continuously visualize the path the stylet 826 takes during insertion into the patient 22.

FIG. 22 shows a medical device 920 which provides a tube changer 926 which is known in the art which is attached to the camera lumen 58/window 60 and separate camera 66. The camera lumen 58/window 60 and separate camera 66 are like that of the previous embodiments of the devices 220, 320, 420, 420', 520, 620, 720, 820 and the specifics are not repeated herein.

As is known in the art, tube changers are used to change one endotracheal tube for another endotracheal tube. The tube changer 926 is formed of an elongated, relatively stiff but compliant plastics material and is preferably formed by extrusion. The tube changer 926 has a proximal end, a distal end and a central passageway therethrough. The proximal end of the changer 926 is capped with a connector 930 that can be connected to a ventilator.

The camera lumen 58 is attached to the tube changer 926 by suitable means, such as ultrasonic welding or a dovetail joint between the camera lumen 58 and the tube changer 926, such that the tube changer 926 and the camera lumen 58 are side-by-side. The distal ends of the tube changer 926 and the camera lumen 58 preferably terminate at the same point. As is described herein with respect to the other embodiments, the camera 66 is removably placed in this sealed camera lumen 58 to continuously visualize the path the tube changer 926 takes during insertion into the patient 22.

In use, the medical professional feeds the medical device 920 through the endotracheal tube that is to be removed until the distal end of the medical device 920 is positioned proximate to the carina 84 of the patient 22. The medical professional can see the distal end of the endotracheal tube as the camera 66 passes thereby and can see the carina 84. The medical professional uses the camera 66 to constantly visualize the tissues and to determine when the distal end of the medical device 920 is proximate to the carina 84. The connector 930 is removed from the medical device 920 and the endotracheal tube is then removed from the patient 22. If necessary, the patient 22 can be ventilated through the medical device 920 by attaching the connector 930 to the tube changer 926 and attaching a ventilator to the connector 930. In order to insert the new endotracheal tube, the connector 930 is removed from the tube changer 926. The new endotracheal tube (which may be one of the endotracheal tube shown in FIGS. 13-17B) is fed over the medical device 920 and into the trachea of the patient 22. The medical device 920 is then pulled proximally. Once the distal end of the new endotracheal tube is sighted using the camera 66, the medical professional must also be able to see the carina 84 to ensure that the new endotracheal tube was not inserted too far into the trachea (if the endotracheal tube is positioned too deeply, the endotracheal tube can abut the carina 84, or can be positioned in one of the bronchus of the patient 22). The new endotracheal tube can be repositioned at this time if necessary using the camera 66 for the proper positioning. Once the new endotracheal tube is properly positioned, the medical device 920 is removed from the new endotracheal tube. The camera 66 can be removed from the camera lumen 58 and placed into the new endotracheal tube as discussed herein.

The tube changer 926 may have graduation marks 932 thereon between the ends which may be used to double check the position of the new endotracheal tube in accordance with the known Seldinger technique. Since the camera 66 provides visual confirmation of the correct placement of the endotracheal tube, the graduations marks 932 are not necessary.

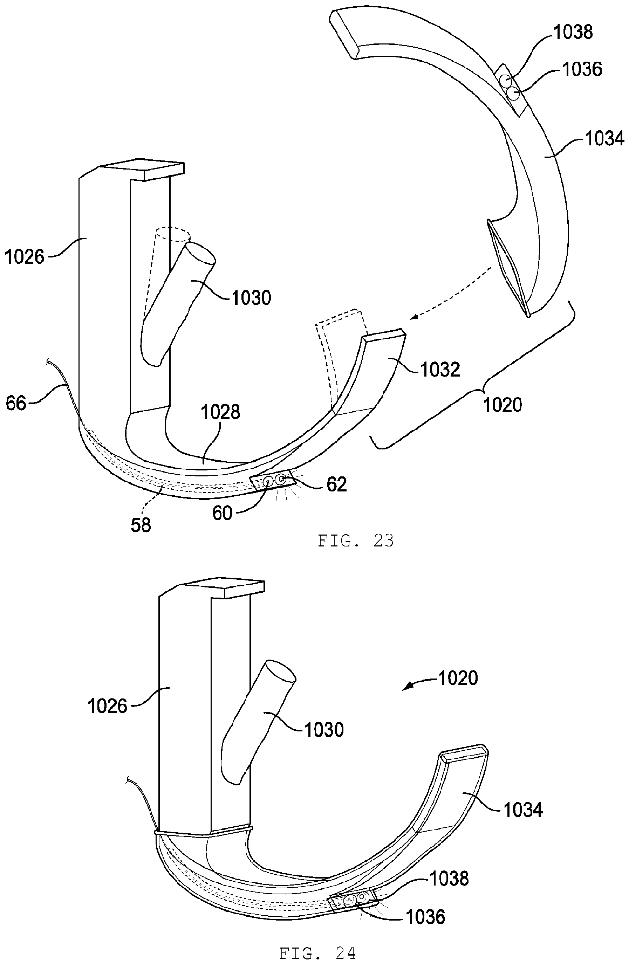

FIGS. 23 and 24 show a medical device 1020 which is used to manipulate the position of the epiglottis 74 of the patient 22 for intubation purposes which is similar to a medical device commonly sold under the trademark GLIDESCOPE® which is owned by Verathon Medical (Canada) ULC. The medical device 1020 has a handle 1026 and curved body 1028 extending from one end of the handle 1026. The handle 1026 has a finger grip handle 1030 extending therefrom at a forty-five degree angle. The finger grip handle 1030 can be flexed relative to the handle 1026. A tip 1032 is provided at the opposite end of the body 1028 and can be flexed relative to the body 1028. A mechanism (not shown), which is known in the art, is embedded in the medical device 1020 and connects the finger grip handle 1030 to the tip 1032. When the medical device 1020 is held by a medical professional, the handle 1 026 seats in the palm of the medical professional and the fingers of the medical professional wrap around the finger grip handle 1030. When the medical professional squeezes his/her fingers, the finger grip handle 1030 moves toward the handle 1026 and this causes the internal mechanism to move the tip 1032 toward the handle 1026. This is known in the prior art.

The prior art medical device 1020 has been modified in two respects. First, integral camera lumen 58/window 60 and LED lights 62 are provided. The integral camera lumen 58 extends along a section of the body 1028. The window 60 is sealed to the camera lumen 58 to prevent the entry of fluids and other matter into the camera lumen 58. This camera lumen 58 terminates at approximately the midpoint of the body 1028. Second, a disposable sleeve 1034 which conforms to the shape of the tip 1032 and body 1028 is provided. The sleeve 1034 is formed of a thin plastic material. The sleeve 1034 has apertures 1036, 1038 therethrough to remove any obstruction from the view of the camera 66 and to allow the lights 62 to shine therethrough in an unobstructed manner. The lights 62 can be incorporated into the sleeve 1034 instead of the body 1028.

In use, the camera 66 is inserted into the camera lumen 58 and the sleeve 1034 covers the tip 1032 and body 1028. The apertures 1036, 1038 in the sleeve 1034 align the window 60 and the lights 62. The medical professional inserts the medical device 1 020 into the mouth of the patient 22 and the tip 1032 enters into the vallecula 110. The body 1028 generally mirrors the shape of the patient's tongue 76. The sleeve 1034 prevents the patient's tissues and secretions from contacting the remainder of the medical device 1020. The handle 1026 seats in the palm of the medical professional and the fingers of the medical professional wrap around the finger grip handle 1030. The entry of the tip 1032 into the mouth of the patient 22 is continuously visualized by the camera 66 in the sealed camera lumen 58. Once the tip 1032 is properly positioned in the vallecula 110, the medical professional's fingers are squeezed and the finger grip handle 1030 moves toward the handle 1026. This causes the internal mechanism in the body 1028 to move the tip 1032 toward the handle 1026. The tip 1032 engages the patient's tongue 76 and pulls the tongue 76 proximally toward the outside of the mouth of the patient 22. As a result, the epiglottis 74 is also pulled proximally to further open the airway of the patient 22.

With the epiglottis 74 pulled proximally, the medical professional inserts the medical device 420, 420', 520 in the patient's throat as described herein. The camera 66 can be removed from the medical device 1020 and inserted into the medical device 420, 420', 520.

In all of the devices 220, 320, 420, 420', 520, 620, 720, 820, 920, 1020 described herein, the same camera 66 can be easily slid into and removed from all of the sealed camera lumens 58. As a result, the camera 66, which is an expensive component, can be used in multiple different devices such as those as shown (or other devices which have such a sealed camera lumen) by removing it from one device and inserting it into another device. Since the camera lumen 58 is sealed, it is not necessary to sterilize the camera 66 between uses as the camera 66 does not come into contact with the tissues and/or secretions of the patient 22.

While specific lumens (camera lumen, transmission lumen) are shown and described with respect to each of the embodiments, it is to be understood that other lumens can also be provided each of the embodiments. Such other lumens could be used for insertion of other tools into the patient 22, for the providing oxygen to the patient 22, for suctioning fluids from the patient 22 and the like.

A transmission lumen 68 can be used in any of the devices 220, 320, 420, 420', 520, 620, 720, 820, 920, 1020. If such a transmission lumen 68 is provided, a speaker (not shown) can included to audibilize the breath and heart sounds from the patient 22 transmitted from the transmission lumen 68. The sound from the transmission lumen 68 can be magnified and externalized to the devices. The speaker can be disseminated electronically. The speaker can be provided in any one of the lumens or tubes, or suitably connected to the lumens or tubes.

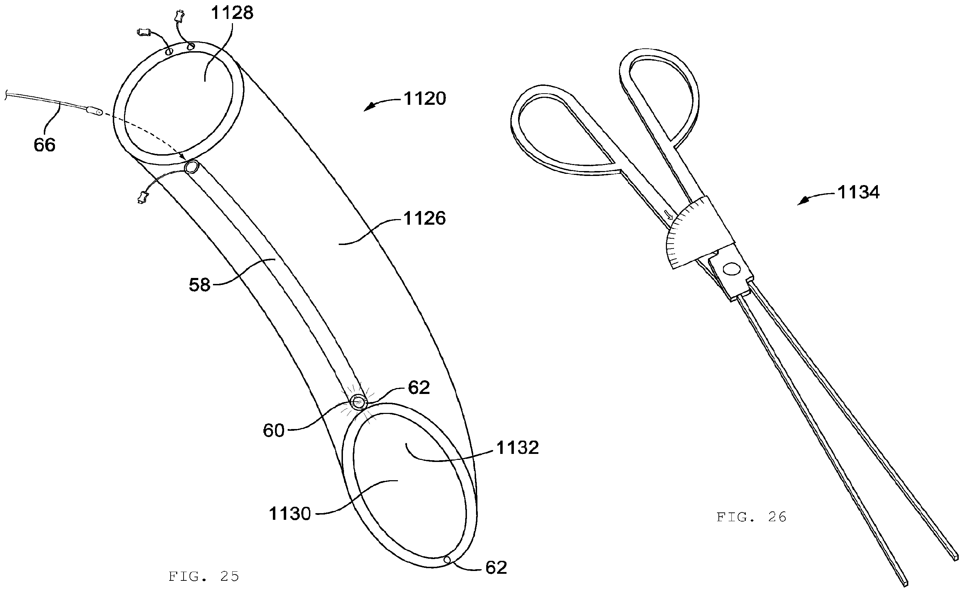

FIGS. 25 and 26 show a medical device 1120 which is allows a medical professional, such as an obstetrician/gynecologist (OB/GYN), to easy view the cervix of a patient to determine the amount of dilation of the cervix. The medical device 1120 includes a cylindrical tube 1126 which has the camera lumen 58/window 60 attached thereto. The separate camera 66 is insertable and removable from the camera lumen 58. The camera lumen 58/window 60 and separate camera 66 are like that of the previous devices 220, 320, 420, 420', 520, 620, 720, 820, and the specifics are not repeated herein.

The tube 1126 has a proximal open inlet 1128 (at the end closest to the medical professional), an opposite distal outlet 1130 (at the end furthest away from the medical professional during use) and a central passageway 1132 extending therethrough. The tube 1126 may be curved along its length, or it may be straight. The tube 1126 is formed of a relatively stiff but compliant plastics material and is preferably formed by extrusion.

The camera lumen 58 is affixed, such as by ultrasonic welding, to the tube 1126, such that the tube 1126 and the camera lumen 58 are side-by-side. The camera lumen 58 can be on the external surface or external surface of the tube 1126. The proximal and distal ends of the tube 1126 and the camera lumen 58 preferably are at the same points. As is described herein with respect to the other embodiments, the camera 66 is removably placed in this sealed camera lumen 58. As in the previous devices, an LED light 62 can be provided at the distal end 1130 of the tube 1132 or camera lumen 58 to illuminate the tissues, or in the camera 66 itself.

In use, the medical professional inserts the medical device 1120 into the patient's vagina such that the cervix can be seen. The camera 66 provides constant visualization of the cervix and tissues during insertion and use of the medical device 1120 in the patient and the medical professional can be assured that the medical device 1120 is being properly positioned in the patient with limited trauma to the patient. The constant visualization of the tissues of the patient can make earlier diagnoses of issues, for example, but not limited to, secretions, tumors, bleeding, and abnormal anatomy, as well as other potentially harmful effects to the patient.

After the medical device 1120 is positioned, the medical professional can use a tool, such as the tool 1134 shown in FIG. 26, to measure the amount of dilation of the cervix. The medical device 1120 remains in place during the labor. The camera 66 provides a constant stream of information to the medical professional regarding the amount of dilation of the patient.

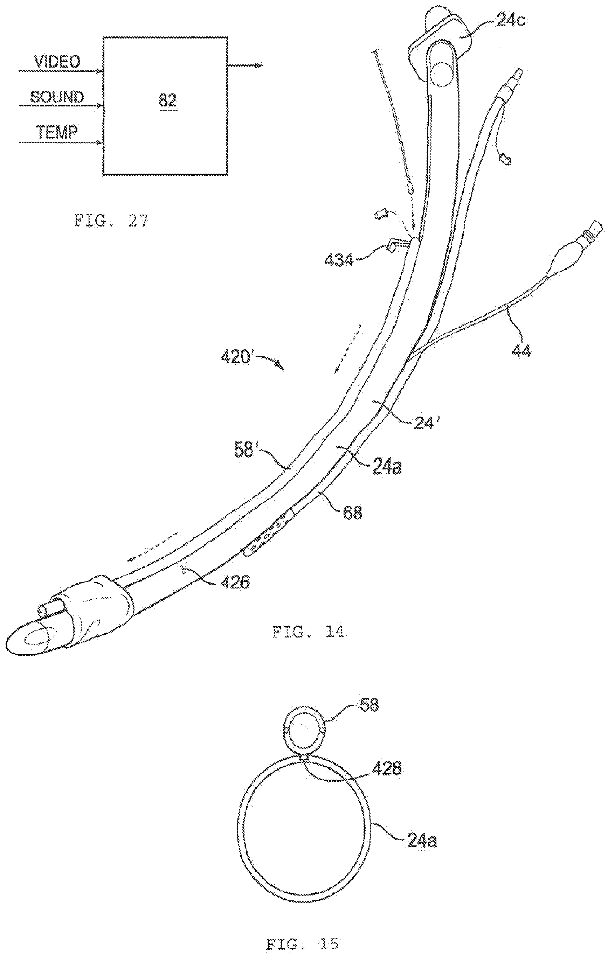

In any of the devices 220, 320, 420, 420', 520, 620, 720, 820, 920, 1020, 1120, the video information from the camera 66 and/or the information from the transmission lumen 68 are transmitted to a microprocessor 82, FIG. 27, via appropriate means, such as wires, wireless, Bluetooth, etc., which in turn can transmit the information to another computer, mobile devices, a mobile station and the like, via appropriate means, such as wires, wireless, Bluetooth, etc., and then this information can be accessed by appropriate personnel. This microprocessor 82 can be on-site where the procedure is being performed or can be remote from the procedure site. For example, the information can be supplied to the nurses' station and the nurse on duty will be able to instantly know if the patient 22 is breathing by the visual confirmation that the vocal folds are opening and closing and by hearing breath and heart sounds. The medical professional will be able to interpret the depth of anesthesia by looking at the rhythmic movement of the vocal folds as well as other diagnoses previously mentioned. Other medical personnel can be hundreds of miles away and still be able to monitor, advise, confirm, and diagnose without the patient 22 being in close physical proximity to that medical personnel. Since the camera 66 is constantly operating, medical personnel can tell at any time if the patient 22 is properly ventilated/intubated and is breathing.

In any of the devices 220, 320, 420, 420', 520,620,720, 820,920, 1020, 1120, the LED light can be provided at the distal end of the device 220, 320, 420, 420', 520, 620, 720, 820, 920, 1020, 1120, in camera lumen 58, or in the camera 66 itself to illuminate the tissues. In addition, multiple LED lights can be provided in each of the 220, 320, 420, 420', 520, 620, 720, 820, 920, 1020, 1120 and can be located on different parts of the devices 220, 320, 420, 420', 520, 620, 720, 820, 920, 1020, 1120.

## Claims

1. A medical device for insertion into a cavity of a patient comprising:
an endotracheal tube (24) for use in intubating a patient comprising a wall (24a) having a proximal end and a distal end, and a passageway extending between said proximal and distal ends, wherein after the patient is intubated, the endotracheal tube (24) provides continuous visualization;
a connector (24c) at said proximal end of said wall (24a) for connecting said endotracheal tube (24) to a ventilating machine;
an inflatable cuff (24b) attached proximate to the distal end of said wall (24a), and a mark (426) provided on said wall (24a) proximal of said inflatable cuff (24b);
a camera lumen which is formed by a flexible plastic tube (58) having a sealed window (60) at one end thereof, said camera lumen (58) attached to said wall (24a) of said endotracheal tube (24), and said sealed window (60) being proximate to said distal end of said wall (24a); and
a separate camera (66) insertable into said camera lumen (58), said camera (66) being removable from said camera lumen (58).

2. The medical device of claim 1, wherein said mark (426) is preferably one inch proximal of said inflatable cuff (24b).

3. The medical device of claim 1, wherein said camera lumen (58) is slidably connected to said endotracheal tube (24).

4. The medical device of claim 1, further including a small cylindrical tube (430) affixed to said wall (24a) and provided between said inflatable cuff (24b) and said wall (24a), said small cylindrical tube (430) having a passageway therethrough, said camera lumen (58) can be slid into and out of said small cylindrical tube (430).

5. The medical device of claim 4, further including a tongue formed on one of said camera lumen (58) and said wall (24a) of said endotracheal tube (24) and a groove formed on the other of said camera lumen (58) and said wall (24a) to provide said slidable connection.

6. The medical device of claim 1, wherein said endotracheal tube (24) is a dual branch endotracheal tube (524).

7. The medical device of claim 1, further comprising a transmission lumen (68) seated within said passageway and extending from an end thereof, said transmission lumen (68) used for transmitting breath and heartbeat sounds from the patient to a medical professional.

8. The medical device of claim 1, wherein the device further comprises: an intubating tube (326) formed of a body (330) having a proximal end and a distal end, an elongated recess (334) formed in said body (330) having a proximal end, a distal end and a length, said recess (334) generally extending from said proximal end of said body to said distal end of said body (330);
a sleeve (328) connected to said intubating tube (326), said sleeve (328) being C-shaped such that an elongated slot is formed, said sleeve (328) being rotatable relative to said intubating tube (326), said sleeve (328) having a length which is less than the length of the recess; wherein the endotracheal tube (24) is inserted within the recess (334);
a camera lumen which is formed by a flexible plastic tube (58) having a sealed window (60) at one end thereof, said camera lumen (58) attached to said intubating tube (326), said sealed window (60) being positioned proximate to said distal end of said recess (334);
a separate camera (66) insertable into said camera lumen (58), said camera (66) being removable from said camera lumen (58).

9. The medical device of claim 8, wherein said body (330) has a generally conical tip (332) at said distal end, said tip having a weight therein.

10. The medical device of claim 8, wherein said recess (334) is curved along its length such that in cross-section said recess is generally arcuate and forms a proximal curved ramp surface.

## Patentansprüche

1. Medizinische Vorrichtung zum Einführen in eine Höhle eines Patienten, umfassend:
einen Endotrachealtubus (24) zur Verwendung bei der Intubation eines Patienten, umfassend eine Wand (24a) mit einem proximalen Ende und einem distalen Ende und einem Durchgang, der sich zwischen dem genannten proximalen und distalen Ende erstreckt, wobei der Endotrachealtubus (24) nach Intubation des Patienten kontinuierlich Visualisierung bereitstellt;
einen Verbinder (24c) an dem genannten proximalen Ende der genannten Wand (24a) zum Verbinden des genannten Endotrachealtubus (24) mit einem Beatmungsgerät;
eine aufblähbare Blockmanschette (24b), die nahe dem distalen Ende der genannten Wand (24a) angebracht ist, und eine an der genannten Wand (24a) proximal der genannten aufblähbaren Blockmanschette (24b) bereitgestellte Markierung (426);
ein Kameralumen, das von einem biegsamen Plastikrohr (58) gebildet wird, das an einem Ende davon ein dicht verschlossenes Fenster (60) aufweist, wobei das genannte Kameralumen (58) an der genannten Wand (24a) des genannten Endotrachealtubus (24) angebracht ist und das genannte dicht verschlossene Fenster (60) nahe dem genannten distalen Ende der genannten Wand (24a) ist; und
eine separate Kamera (66), die in das genannte Kameralumen (58) einsetzbar ist, wobei die genannte Kamera (66) aus dem genannten Kameralumen (58) entfernbar ist.

2. Medizinische Vorrichtung nach Anspruch 1, wobei die genannte Markierung (426) vorzugsweise ein Zoll proximal der genannten aufblähbaren Blockmanschette (24b) ist.

3. Medizinische Vorrichtung nach Anspruch 1, wobei das genannte Kameralumen (58) gleitfähig mit dem genannten Endotrachealtubus (24) verbunden ist.

4. Medizinische Vorrichtung nach Anspruch 1, die ferner einen kleinen zylindrischen Tubus (430) beinhaltet, der an der genannten Wand (24a) befestigt ist und zwischen der genannten aufblähbaren Blockmanschette (24b) und der genannten Wand (24a) bereitgestellt ist, wobei der kleine genannte zylindrische Tubus (430) einen Durchgang durch ihn hindurch aufweist, wobei das genannte Kameralumen (58) in den und aus dem genannten kleinen zylindrischen Tubus (430) geschoben werden kann.

5. Medizinische Vorrichtung nach Anspruch 4, die ferner eine Feder, die an einem von dem genannten Kameralumen (58) und der genannten Wand (24a) des genannten Endotrachealtubus (24) gebildet ist, und eine Nut, die an dem anderen von dem genannten Kameralumen (58) und der genannten Wand (24a) gebildet ist, beinhaltet, um die genannte gleitfähige Verbindung bereitzustellen.

6. Medizinische Vorrichtung nach Anspruch 1, wobei der genannte Endotrachealtubus (24) ein zweiarmiger Endotrachealtubus (524) ist.

7. Medizinische Vorrichtung nach Anspruch 1, die ferner ein Übertragungslumen (68) umfasst, das in dem genannten Durchgang sitzt und sich von einem Ende davon erstreckt, wobei das genannte Übertragungslumen (68) zum Übertragen von Atmungs- und Herzschlaggeräuschen vom Patienten zu einer medizinischen Fachperson verwendet wird.

8. Medizinische Vorrichtung nach Anspruch 1, wobei die Vorrichtung ferner Folgendes umfasst: einen Intubationstubus (326), der von einem Körper (330) mit einem proximalen Ende und einem distalen Ende gebildet wird, eine in dem genannten Körper (330) gebildete längliche Aussparung (334) mit einem proximalen Ende, einem distalen Ende und einer Länge, wobei die genannte Aussparung (334) sich allgemein von dem genannten proximalen Ende des genannten Körpers zu dem genannten distalen Ende des genannten Körpers (330) erstreckt;
eine Hülle (328), die mit dem genannten Intubationstubus (326) verbunden ist, wobei die genannte Hülle (328) C-förmig ist, so dass ein länglicher Spalt gebildet wird, wobei die genannte Hülle (328) relativ zu dem genannten Intubationstubus (326) drehbar ist, wobei die genannte Hülle (328) eine Länge aufweist, die kleiner als die Länge der Aussparung ist; wobei der Endotrachealtubus (24) in die Aussparung (334) eingesetzt wird;
ein Kameralumen, das von einem biegsamen Plastikrohr (58) gebildet wird, das an einem Ende davon ein dicht verschlossenes Fenster (60) aufweist, wobei das genannte Kameralumen (58) an dem genannten Intubationstubus (326) angebracht ist, wobei das genannte dicht verschlossene Fenster (60) nahe dem genannten distalen Ende der genannten Aussparung (334) positioniert ist;
eine separate Kamera (66), die in das genannte Kameralumen (58) einsetzbar ist, wobei die genannte Kamera (66) aus dem genannten Kameralumen (58) entfernbar ist.

9. Medizinische Vorrichtung nach Anspruch 8, wobei der genannte Körper (330) an dem genannten distalen Ende eine allgemein kegelförmige Spitze (332) aufweist, wobei die genannte Spitze ein Gewicht in ihr aufweist.

10. Medizinische Vorrichtung nach Anspruch 8, wobei die genannte Aussparung (334) entlang ihrer Länge gekrümmt ist, so dass die genannte Aussparung im Querschnitt allgemein bogenförmig ist und eine proximal gekrümmte Rampenfläche bildet.

## Revendications

1. Dispositif médical destiné à être inséré jusque dans une cavité d'un patient comprenant :
un tube endotrachéal (24) destiné à être utilisé dans l'intubation d'un patient comprenant une paroi (24a) ayant une extrémité proximale et une extrémité distale, et une voie de passage s'étendant entre lesdites extrémités proximale et distale, dans lequel une fois que le patient est intubé, le tube endotrachéal (24) fournit une visualisation continue ;
un connecteur (24c) au niveau de ladite extrémité proximale de ladite paroi (24a) destiné à connecter ledit tube endotrachéal (24) à une machine de ventilation ;
un manchon gonflable (24b) attaché à proximité de l'extrémité distale de ladite paroi (24a), et une marque (426) fournie sur ladite paroi (24a) proximale par rapport audit manchon gonflable (24b) ;
une lumière de caméra qui est formée par un tube en plastique souple (58) ayant une fenêtre étanche (60) au niveau d'une extrémité de celui-ci, ladite lumière de caméra (58) attachée à ladite paroi (24a) dudit tube endotrachéal (24), et ladite fenêtre étanche (60) étant à proximité de ladite extrémité distale de ladite paroi (24a) ; et
une caméra distincte (66) pouvant être insérée jusque dans la lumière de caméra (58), ladite caméra (66) pouvant être retirée de ladite lumière de caméra (58).

2. Dispositif médical selon la revendication 1, dans lequel ladite marque (426) est de préférence proximale à un pouce dudit manchon gonflable (24b).

3. Dispositif médical selon la revendication 1, dans lequel ladite lumière de caméra (58) est connectée de manière coulissante audit tube endotrachéal (24).

4. Dispositif médical selon la revendication 1, incluant en outre un petit tube cylindrique (430) fixé à ladite paroi (24a) et fourni entre ledit manchon gonflable (24b) et ladite paroi (24a), ledit petit tube cylindrique (430) comportant une voie de passage à travers celui-ci, ladite lumière de caméra (58) peut être glissée dans ledit petit tube cylindrique (430) et hors de celui-ci.

5. Dispositif médical selon la revendication 4, incluant en outre une languette formée sur l'une de ladite lumière de caméra (58) et de ladite paroi (24a) dudit tube endotrachéal (24) et une rainure formée sur l'autre de ladite lumière de caméra (58) et de ladite paroi (24a) pour fournir ladite connexion coulissante.

6. Dispositif médical selon la revendication 1, dans lequel ledit tube endotrachéal (24) est un tube endotrachéal à deux branches (524).

7. Dispositif médical selon la revendication 1, comprenant en outre une lumière de transmission (68) logée au sein de ladite voie de passage et s'étendant à partir d'une extrémité de celle-ci, ladite lumière de transmission (68) utilisée pour transmettre des sons de respiration et de battement cardiaque du patient à un professionnel de santé.

8. Dispositif médical selon la revendication 1, dans lequel ledit dispositif comprend en outre : un tube d'intubation (326) formé d'un corps (330) ayant une extrémité proximale et une extrémité distale, un renfoncement allongé (334) formé dans ledit corps (330) ayant une extrémité proximale, une extrémité distale et une longueur, ledit renfoncement (334) s'étendant de manière générale de ladite extrémité proximale dudit corps à ladite extrémité distale dudit corps (330) ;
un manchon (328) connecté audit tube d'intubation (326), ledit manchon (328) étant en forme de C de sorte qu'une fente allongée est formée, ledit manchon (328) étant capable de rotation par rapport audit tube d'intubation (326), ledit manchon (328) ayant une longueur qui est inférieure à la longueur du renfoncement ; dans lequel le tube endotrachéal (24) est inséré au sein du renfoncement (334) ;
une lumière de caméra qui est formée par un tube en plastique souple (58) ayant une fenêtre étanche (60) au niveau d'une extrémité de celui-ci, ladite lumière de caméra (58) attachée audit tube d'intubation (326), ladite fenêtre étanche (60) étant positionnée à proximité de ladite extrémité distale dudit renfoncement (334) ;
une caméra distincte (66) pouvant être insérée jusque dans ladite lumière de caméra (58), ladite caméra (66) pouvant être retirée de ladite lumière de caméra (58).

9. Dispositif médical selon la revendication 8, dans lequel ledit corps (330) a une pointe conique de manière générale (332) au niveau de ladite extrémité distale, ladite pointe ayant un poids dans celle-ci.

10. Dispositif médical selon la revendication 8, dans lequel ledit renfoncement (334) est courbé le long de sa longueur de sorte qu'en coupe droite ledit renfoncement est arqué de manière générale et forme une surface de rampe courbée proximale.
